# EUROPEAN PATENT APPLICATION

(11) **EP 4 811 369 A1**
(43) Date of publication of application: **23.09.2026**
(21) Application number: 25185515.1
(22) Date of filing: 26.06.2025
(51) Int. Cl.: G16H 10/60, A61N 5/00, G16H 20/40, G16H 40/60, H04L 9/00, A61B 6/00

(54) **MEDICAL SECURITY MANAGEMENT SYSTEM AND METHOD**

(30) Priority: 19.03.2025 TW 114110224
(71) Applicant: Heron Neutron Medical Corp., Zhubei City, Hsinchu County (TW)
(72) Inventor: CHEN, Wei-Lin, Zhubei City, Hsinchu County (TW); HUANG, Wei-Lun, Zhubei City, Hsinchu County (TW); CHEN, Wen-Tzu, Zhubei City, Hsinchu County (TW); CHENG, Hsin Tien, Zhubei City, Hsinchu County (TW)
(74) Representative: Patentship Patentanwaltsgesellschaft mbH

(57) **Abstract**

A medical security management system (100) and method, including: a clinical control terminal (110), further including a clinical security control module (112); a neutron beam control terminal (120), connected to the clinical control terminal (110), controlling the operation of the accelerator terminal (200), wherein the neutron beam control terminal (120) further includes an accelerator security control module (121). The system (100) further including a consensus mechanism module (130), storing at least one consensus mechanism. When the clinical control terminal (110) transmits a neutron beam generation request to the neutron beam control terminal (120), the accelerator security control module (121) and the clinical security control module (112) respectively generate and transmit a first key and a second key to the consensus mechanism module, thereby the consensus mechanism module drive the accelerator terminal to generate a neutron beam if the first key (1) and the second key (2) meet the requirement of the consensus mechanism.

## Description

### TECHNICAL FIELD OF THE INVENTION

The present invention relates to medical security management systems and methods, in particular, it relates to medical security management systems and methods that improve the security management process of neutron beam generation timing in radiotherapy technology.

### BACKGROUND OF THE INVENTION

Boron neutron capture therapy (BNCT) is an innovative radiation therapy for cancer by boron-containing drugs (such as amino acids serving as carriers for the boron element), these drugs are designed to selectively accumulate in the tumor cells of patients with minimal absorption in normal cells. After the patient is exposed to an epithermal neutron beam, the tumor cells that have accumulated the boron-containing drug are more likely to absorb the neutrons than normal healthy cells, consequently, a nuclear reaction occurs when the neutrons interact with the boron-containing drugs, producing lithium nuclei (⁷Li) and alpha particles. The generated alpha particles and lithium nuclei (⁷Li) atoms have an extremely short range (about 5~9 micrometers) and can destroy tumor cells. This range is roughly equal to the diameter of a cell, allowing the energy to be concentrated within the tumor cells, causing minimal damage to the surrounding healthy tissues. Therefore, BNCT treatment offers advantages such as high selectivity for tumor sites, low toxicity, fewer treatment sessions compared to traditional radiation therapy, less burden on the body, and a faster recovery period.

For the aforementioned Boron Neutron Capture Therapy (BNCT), the generation of neutron beam is a of crucial procedure. Since the generation of neutron beam belongs to the behavior of radiation production, such procedure requires particular security management mechanism to ensure each step is carried out systematically for reducing the risk associated with the neutron beam from generation to output. In general, the architecture of BNCT medical system can be divided into two major subsystems according to the tasks, namely the neutron beam generation system and the clinical treatment system. The former typically includes equipment such as accelerators and cooling water modules and is operated by the radiation control terminal. The latter is managed by the medical control terminal and is usually located in the control room used by medical staff, wherein the clinical treatment system include the outlet of neutron beam, patient positioning devices, radiation safety control equipment, and the patient treatment area, primarily operated by medical radiologic technologists, who are responsible for monitoring and controlling the equipment within the clinical treatment system to ensure that the radiation dose condition aligns with the treatment plan.

As mentioned above, the radiation security control equipment of clinical treatment system generally includes the radiation detection devices, alarm devices, networked servers, and other equipment to provide the on-site medical personal with improved therapy efficiency and radiation security during the process in the currently BNCT industry. In general, the radiation detection devices are disposed in medical facilities, such as the accelerator equipment rooms, medical staff control rooms, and patient therapy areas to detect the environmental radiation amount in the various areas of medical facilities. In addition, the alarm devices (generally alarm bells, light signs, or voice alarms) are disposed in the medical facilities to send alarms and prompt the situation in response to the amount of the environmental radiation. Finally, the networked servers generally connect the radiation detection devices and alarm devices to comprehensively determine whether to issue the corresponding alarms based on the operation of the clinical therapy system, neutron beam generation system, radiation detection devices, alarm devices, or the amount of captured environmental radiation. This achieves the aforementioned reduction of risks from the generation to the output of the neutron beam.

According to the conventional technology, despite the networked severs, alarm devices, and other related facilities that have been developed, the security core of the BNCT medical system still fundamentally relies on the mechanism and regulations of neutron beam generation, as well as the approach of interaction with each control terminal, system architecture, and the corresponding method steps. However, the prior art still lacks of the comprehensive discussion of the neutron beam security mechanism at each control terminal in the current time, especially in the period of the neutron beam generation process. Consequently, there are still existing deficiencies in interaction timing and the corresponding control part of the two subsystems, i.e. the neutron beam generation system and clinical therapy system. In the aforementioned circumstance, the neutron beam generation mechanism is still concentrated under the control of the neutron beam generation system. The lack of control authority of the clinical therapy system for the neutron beam generation causes potential risks of the operation processes of the BNCT medical system, therefore, the improvement of security management structures is needed.

### BRIEF SUMMARY OF THE INVENTION

Based on the reasons provided above, the purpose of the present invention is to provide an innovative medical security management system and method, aiming to improve the security of the BNCT medical system. The present invention aims to clarify the bidirectional information transmission mechanism to resolve the communication and control problems during human communication of the existing BNCT medical system through the well-defined authorization scheme. The deficiency in the prior art may cause information to be unclear during the system's operation and lead to the delay of key decisions, which is especially dangerous in emergencies. The improvement techniques include: (1) the requesting mechanism of neutron beam and authority allocation of control. In particular, when a "neutron beam generation request" is proposed, this request signal is sent to the neutron beam control end by the clinical control end. After confirming that all the equipment is operating normally, the neutron beam control end authorizes the clinical control end to enable the neutron beam, and finally enables the accelerator end to control the accelerator operation to complete the generation of the neutron beam. (2) Rapid response to the abnormal situations. If the patients or equipment are abnormal, the "pause" or "turn off" request of the neutron beam generation can be directly sent by any terminal without waiting for the delays caused by the transmission delays between the terminals, ensuring that the risk of radiation leakage can be responded immediately. Therefore, the two improvement directions proposed above may ensure that each terminal and subsystem of the BNCT medical system completes the corresponding security inspections and meets the requirements for radiation leakage security management of radiotherapy. Based on the purpose above, the detailed technical approach please refer to the following description.

To achieve the purpose of the present invention, the present invention provides a medical security management system in a broad embodiment which include: a clinical control end (terminal), a neutron beam control end (terminal), and a consensus mechanism module. The clinical control end can be disposed in a therapy control room, and further comprise a clinical security control module to control the operation of the therapy control room. The neutron beam control end is disposed in the radiation control room, coupled to the clinical control end to control the operation of the accelerator end, wherein the neutron beam control end further includes an accelerator security control module. Furthermore, the consensus mechanism module is coupled to the clinical security control module, the accelerator security control module, and the accelerator end, storing the consensus mechanism. When the clinical control end sends a neutron beam generation request to the neutron beam control end, the accelerator security control module and the clinical security control module generate and transmit a first key and a second key respectively to the consensus mechanism module, resulting in the consensus mechanism module driving the accelerator end to generate a neutron beam via the consensus mechanism.

According to the content of the present invention, the medical security management system further includes a neutron beam planning module, coupled to the accelerator security control module, planning a neutron beam plan based on medical requirements and transmitting a neutron beam generation request to the accelerator security control module of the neutron beam control end, providing the neutron beam control end to make an evaluation of whether to approve the neutron beam generation request.

To achieve the purpose of the present invention, the present invention also provides a medical security management method, including the following processes: preparing a neutron beam plan; clinical control end proposes a neutron beam generation request based on the neutron beam plan; an accelerator security control module transmits a first key to a consensus mechanism module; the clinical control end transmits a second key to the consensus mechanism module; the consensus mechanism module allows the execution of the neutron beam plan, driving an accelerator end to generate a neutron beam based on the first key and the second key.

According to the content of the present invention, the medical security management method further includes that the consensus mechanism module starts to drive the accelerator end to generate the neutron beam when the first key and the second key are scheduled simultaneously in the consensus mechanism module.

### BRIEF DESCRIPTION OF THE DRAWINGS

The present invention can be fully understood by reading the subsequent detailed description and examples with references made to the accompanying drawings; however, it should be understood that these are only used as a reference for understanding the application of the present invention, and do not limit the present invention to a specific embodiment, wherein:
FIG. 1 illustrates a system structure of a medical security management system.
FIG. 2A illustrates a detailed system structure of a consensus mechanism module.
FIG. 2B shows the conditions of neutron beam generation in an embodiment of the present invention.
FIG. 3 is a flowchart which illustrates a medical security management method.
FIG. 4 is a flowchart which illustrating the medical security management method.
FIG. 5A illustrates the information included in a neutron beam plan in some embodiments.
FIG. 5B illustrates the information included in the neutron beam plan.

### DETAILED DESCRIPTION OF THE INVENTION

The following description is made to illustrate the general principles of the invention and should not be taken in a limiting sense. The scope of the invention is best determined by reference to the appended claims.

The present invention will be described in detail with the preferred embodiments and aspects so that the reader can fully understand the implementation of these embodiments. However, those skilled in the art should understand that the present invention can be implemented without these details. Furthermore, the present invention can also be used or implemented through other specific embodiments. The various details described in the present specification can also be used based on the different needs, and the various modifications or changes can be performed. Therefore, the present invention will be described in the preferred embodiments and aspects. Such descriptions are to explain the structure of the present invention, and are only used to illustrate but not to limit the scope of claims of the present invention. The terms used in the following description will be interpreted in the broadcast reasonable manner. Those skilled in the art can adjust the structure of the present invention based on the needs of manufacturing or therapeutic applications to meet the needs of actual industry. This is explained in advance.

As used in the present invention, "about", "approximately", or "substantially" shall generally indicate within 20%, preferably within 10%, and more preferably within 5% of a given value or scope. The numerical values given herein are approximate, indicating that the meanings of terms "about", "approximately", or "substantially" may be inferred if there is no explicit illustration. Furthermore, directions, such as "front and rear", "left and right", "up and down" used in the present invention can be understood regarding the description of the drawings. Unless otherwise illustrated in the present invention, after reading the description of the present invention, these can be understood through the context of the description. Finally, for the sake of simplifying the drawings, some commonly used and known structures and components are illustrated simply and schematically in the drawings. For the convenience of viewing, the size of each component in the drawings are not drawn to scale. Furthermore, in the description of the present invention, the method or process orders are only the process orders of a certain method or process, unless otherwise indicated in the description of the present invention specifically. These are only used for clarity and convenience of the illustration, and are not used to illustrate the sequence of method process. Finally, it should be noticed that the terms, such as the time interval, first time, second time, third time, and fourth time, illustrated in the present invention refer to the time when the clinical control end and neutron beam control end theoretically start to send or operate. In practical, there may be a delay in transmission or system execution of the transmission. The scope of delay is also within the scope of interpretation of the present invention. However, after reading the present invention, those skilled in the art can modify or change it in practice based on the delay to meet the needs of applications. This is also explained in advance.

According to an embodiment of the present invention, a medical security management system 100 may be software, hardware, or a combination of software and hardware. If the medical security management system 100 is a software, when its workflow is installed in a computer or multiple computer systems, the computer systems are used for performing the present invention. If the medical security management system 100 is a hardware, it includes the common processing chips, memory, scratch pad memory, display devices, network communication interfaces, operating systems, and applications, which are coupled to each other in common and known methods to perform the calculations, temporary storage, display, and data transmission. Each component of the medical security management system 100 can also be a combination of software and hardware. Since the above structures are commonly knowledge in the art, the details are not reiterated. Furthermore, it should be noticed that each component of the medical security management system 100 and the sub-components of each of the components, including a neutron beam planning module 111, a clinical security control module 112, a monitoring module 113, an accelerator security control module 121, a consensus mechanism module 130, or any other components disclosed herein can be deployed in multiple terminals (such as a clinical control end 110, a neutron beam control end 120, an accelerator end 200) integrally or separately based on the needs of applications, allowing each component of the medical security management system 100 and the sub-components of each of the components can interact with each other through the remote connections without being limited to be deployed nearby. After reading the description of the present invention, those skilled in the art can modify or change based on the needs of applications.

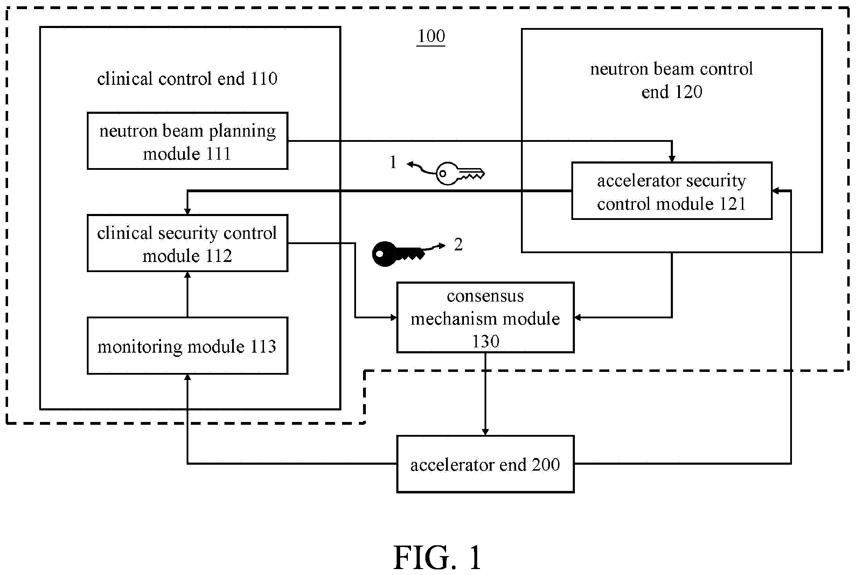

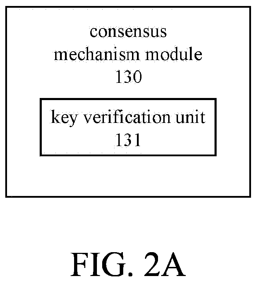

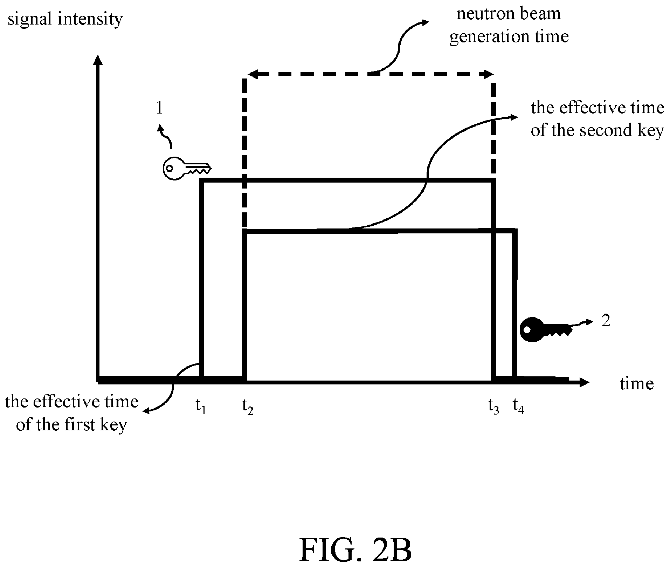

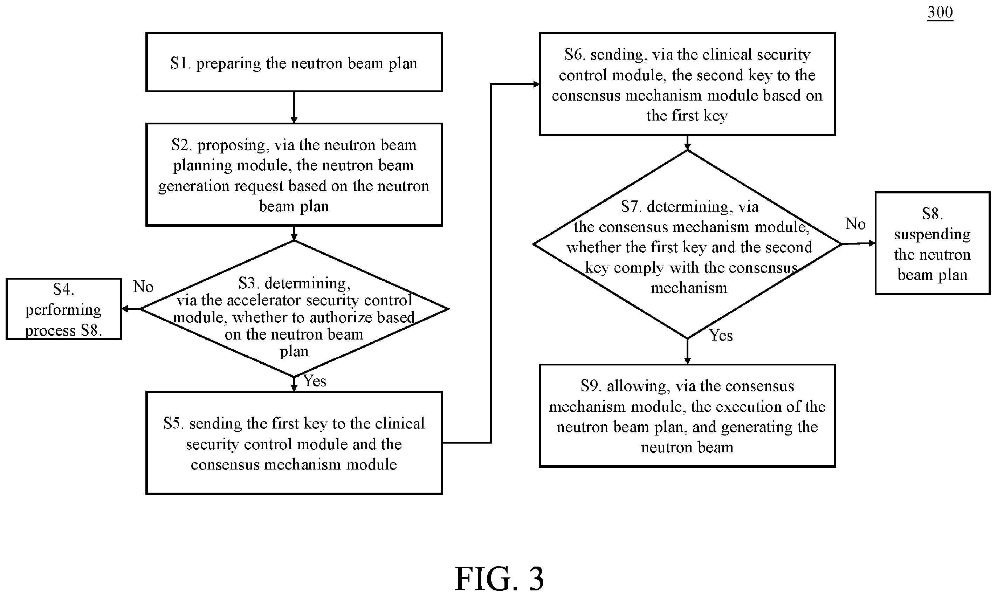

Please refer to FIGS. 1, 2A, and 2B. To solve the problems discussed above, the present invention provides the medical security management system 100, including the clinical control end 110, which includes the clinical security control module 120 to control an operation of the therapy control room; the neutron beam control end 120, coupled to the clinical control end 110 to control an operation of the accelerator end 200, the neutron beam control end 120 further including the accelerator security control module 121; and the consensus mechanism module 130, coupled to the clinical security control module 112, the accelerator security control module 121, and the accelerator end 200, storing at least one consensus mechanism. When the clinical security control module 112 of the clinical control end 110 transmits a neutron beam generation request to the accelerator security control end 121 of the neutron beam control end 112, the accelerator security control module 121 and the clinical security control module 112 will generate and transmit a first key 1 and a second key 2 respectively to the consensus mechanism module 130, thereby the consensus mechanism module 130 drives the accelerator end 200 to generate a neutron beam via the consensus mechanism. Please refer to FIG. 3 for further details. According to an embodiment of the present invention, the FIG. 3 illustrates one of the interactive operation approaches of the clinical security control module 112, the accelerator security control module 121, and the consensus mechanism module 130. In this embodiment, when the accelerator security control module 121 transmits the first key 1 to the clinical security control module 112 and the consensus mechanism module 130 at a first time t₁, the first key 1 includes an effective timestamp, for example, in FIG. 2B, the two timestamps are the first time t₁ and a third time t₃ (i.e., t₁-t₃), which indicate that the effective period (time interval) of the first key 1. When receiving the first key 1, the clinical security control module 112 determines the time required for generating the neutron beam based on the application and therapy needs of control ends (the accelerator security control module 121 is the same), transmitting the second key 2 to the consensus mechanism module 130. This allows an effective period of the second key 2 which start from second time t₂ to fourth time t₄ (i.e., t₂-t₄). When the consensus mechanism module 130 receives the first key 1 and the second key 2, an intersection of effective period is obtained based on the first key 1 and the second key 2. That is, the time interval from the second time t₂ to the third time t₃ (i.e., t₂-t₃) is used as the neutron beam generation time. The time when the first key 1 and the second key 2 are jointly transmitted to the consensus mechanism module 130 which is used as the consensus mechanism between the clinical control end 110 and the neutron beam control end 120. Therefore, the clinical control end 110 and the neutron control end 120 can jointly monitor the neutron beam generated by the accelerator end 200. Accordingly, should an effective time of one of the keys (the first key 1 or the second key 2) expires, the consensus mechanism, which requires both the first key 1 and the second key 2 active simultaneously to drive the neutron beam generation will no longer be satisfied, resulting in the shut down of neutron beam by the accelerator end 200. As a result, the neutron beam generation request can be controlled by the clinical control end 110 and the neutron beam control end 120, and one of the terminals can respond in time without waiting the transmission delay between the terminals if an abnormal situation occurs through the authority allocation mechanism, achieving the propose of the present invention.

According to an embodiment of the present invention, the information carried by the aforementioned neutron beam generation request, the first key 1, and the second key 2 may include identity verification information, communication-related information for the neutron beam generation, the aforementioned timestamps (e.g., first time t₁, second time t₂, third time t₃, and fourth time t₄), and the time intervals indicated by the timestamps (such as the accelerator end 200 is driven to generate the neutron beam at t₂-t₃). The aforementioned information may be, but is not limited to text, audio, video, symbols, digital codes, or the combination thereof. The information mentioned above may be public or non-public. For example, the neutron beam generation request may be a code including the text "request", the corresponding first key 1 may be a code including the text "1^{st} beam on", and the corresponding second key 2 may be a code including the text "2^{nd} beam on". In another embodiment of the present invention, in addition to the above text messages, the information may also include the corresponding audio messages. For example, the voices, which are input by the clinical control end 110 and neutron beam control end 120, and correspond to the neutron beam generation request, the first key 1, and the second key 2. This provides the consensus mechanism with interactive verification between the controlled terminals or modules to achieve the purpose of clarifying the information transmission, the authority allocation of control, and improving the information clarity when the medical security management system 100 is operating.

Please refer to FIGS. 1 and 2A. According to the content of the present invention, wherein the consensus mechanism module 130 further includes a key verification unit 131, configured to verify further interactive verification between the first key 1 and the second key 2. According to an embodiment of the present invention, the first key 1 and the second key 2 include not only the aforementioned communication-related information for the neutron beam generation, timestamps, but also the identity verification information. Wherein the identity verification information may include, but not limit to digital signatures, encryption algorithms (which may be symmetric encryption or asymmetric encryption), and biometric technology (such as fingerprint, facial, or iris recognition) for enhancing the identity confirmation strength of each terminal operator in the medical security management system 100. According to an aspect of the present invention, the medical security management system 100 is mainly used in the joint operation and interactive control of multiple terminals, such as the clinical control end 110, the neutron beam control end 120, and the accelerator end 200 (especially for the triggering moment of the neutron beam generation and shutdown under the multi-terminal verification). This indicates that the medical security management system 100 essentially points to serve as an application for the cross-system integration and remote monitoring of the neutron beam, as the number of interconnected terminals increases, or when the medical security management system 100 is required to be integrated and extended to other medical security management platforms, such as the subsystem (e.g., the electrical medical records) of the BNCT medical system, the security and the degree of freedom of remote deployment (i.e. clinical control end 110, the neutron beam planning module 111, the clinical security control module 112, the monitoring module 113, the neutron beam control end 120, and the accelerator security control module 121) could be improvement via the consensus mechanism stored in the consensus mechanism module 130 through the execution of key verification unit 131 for achieving the purpose of the present invention.

Please refer to FIG. 1. According to the content of the present invention, the medical security management system 100 further includes the monitoring module 113. In an embodiment of the present invention, the monitoring module 113 is installed in the clinical control end 110, coupled to the clinical security control module 112 and the accelerator end 200, configured to detect and monitor a neutron beam generation status (e.g., the dose of neutron beam, the radiation generated in the form of photons by the radioactive material activated by the neutron beam (e.g., Gamma ray), the triggering moment (timing) of neutron beam generation and shutdown) of the accelerator end 200, and the monitoring data of the therapy status of the patient. Then, the monitoring module 113 transmits the monitoring data to the clinical security control module 112, allowing the clinical security control module 112 to determine the time interval of which the second key 2 is effective. Accordingly, the effective timing can be defined by the two timestamps, namely, the second time t₂ and the fourth time t₄. Therefore, the monitoring module 130 enables the entire medical security management system 100 to promptly detect and respond to any abnormal situations during the neutron beam generation process, enhancing the integration of neutron beam generation status and patient treatment conditions among the various subsystems of the BNCT medical system correspond to the clinical control terminal 110.

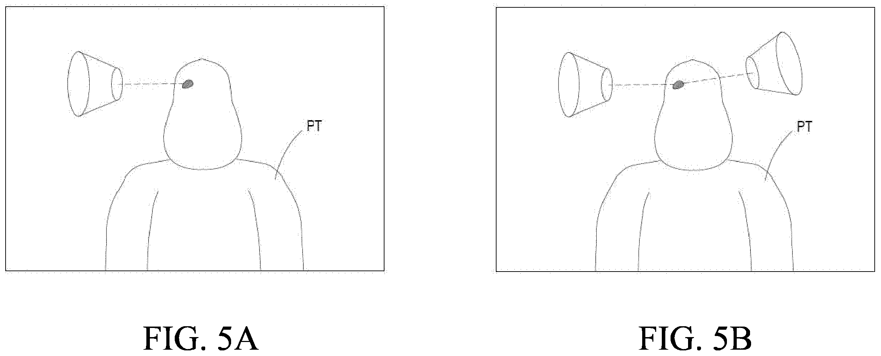

Please refer to FIGS. 1, 5A, and 5B. According to the content of the present invention, the medical security management 100 further includes the neutron beam planning module 111. In an embodiment of the present invention, the neutron beam planning module 111 is installed in the clinical control end 110 to formulate a neutron beam plan based on the medical needs. The neutron beam planning module 111 is coupled to the accelerator security control module 121, transmitting the neutron beam generation request to the accelerator security control module 121, wherein the neutron beam generation request is provided to the neutron beam control end 120 for evaluating whether to approve the neutron beam generation request. Accordingly, the neutron beam plan may include the dose, irradiation time, irradiation intensity, irradiation angle, irradiation position, single field, multiple fields, medical images, conditions to start the irradiation, conditions to end the irradiation, and other information required by the neutron beam for the patient PT. Thus, the accelerator security control module 121 determines whether to transmit the first key 1 to the clinical security control module 112 in accordance with the approved neutron beam generation request under the actual operating conditions, neutron beam quality control (QC)/quality assurance (QA), and other conditions of the accelerator end 200. Therefore, due to the presence of the consensus mechanism module 130, the first key 1, and the second key 2, the communication methods of control ends of the medical security management system 100 are improved, therefore the neutron beam generation request can be directly controlled by the clinical control end 110, achieving the purpose of security improvement in the BNCT medical system of the present invention.

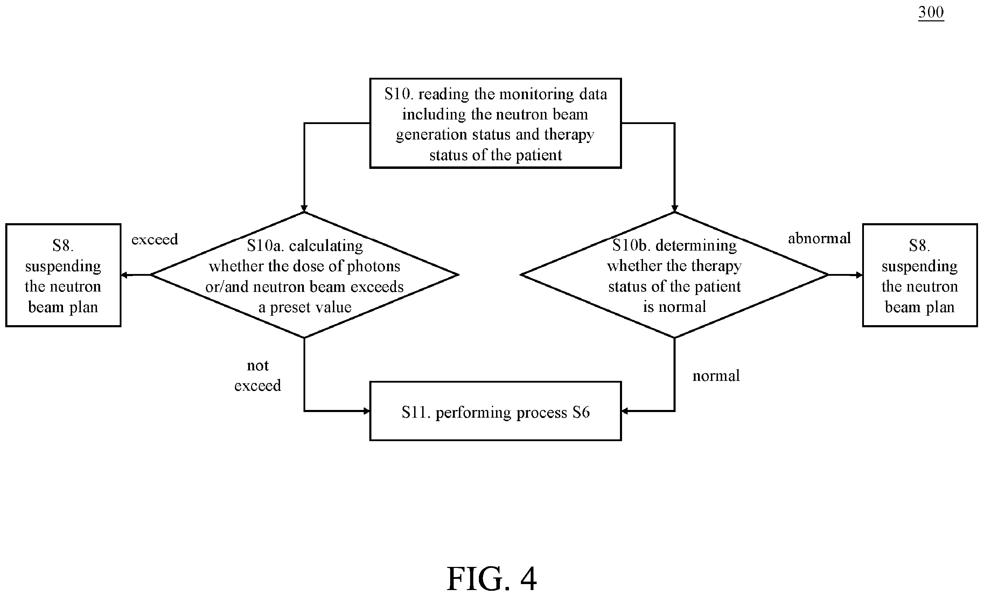

Please refer to FIGS. 2B, 3, and 4. To solve the problems mentioned above, the present invention discusses a medical security management method 300, which includes the following processes. In process S1, the neutron beam plan is prepared. In process S2, the clinical control end 110 proposes the neutron beam generation request based on the neutron beam plan. In process S5, the accelerator security control module 121 of the neutron beam control end 120 transmits the first key 1 to the consensus mechanism module 130 based on the neutron beam generation request. In process S6, the clinical control end 110 transmits the second key 2 to the consensus mechanism module 130. In process S9, the consensus mechanism module 130 allows the execution of the neutron beam plan based on the received first key 1 and the received second key 2, and drives the accelerator end 200 to generate the neutron beam. In the embodiments of the present invention, when the accelerator security control module 121 transmits the first key 1 to the clinical security control module 112 and the consensus mechanism module 130 at the first time t₁, the first key 1 includes an effective timestamp. For example, in FIG. 2B, the two timestamps of the first key 1 indicate that the effective time intervals is between first time t₁ and third time t₃ (i.e., t₁-t₃). Accordingly, the clinical security control module 112 determines the time required for generating the neutron beam based on the application and the therapy needs of control ends (as well as the accelerator security control module 121), transmitting the second key 2 to the consensus mechanism module 130 while receiving the first key 1, allowing the effective time of the second key 2 starts from the second time t₂ to the fourth time t₄ (i.e., t₂-t₄). When the consensus mechanism module 130 receives the first key 1 and the second key 2, the intersection of effective period is obtained based on the first key 1 and the second key 2, in other words, the time interval from the second time t₂ to the third time t₃ (i.e., t₂-t₃) is used as the neutron beam generation time. Consequently, the consensus mechanism based on the time at which both the first key 1 and the second key 2 are transmitted to the consensus mechanism module 130, making the joint monitoring neutron beam generated by the accelerator end 200 through the clinical control end 110 and the neutron control end 120. When the effective time of one of the keys (the first key 1 or the second key 2) expires, the neutron beam will be shut down by the accelerator end 200 due to the consensus mechanism requires both the first key 1 and the second key 2 to be active simultaneously in order to enable neutron beam generation.

Please refer to FIGS. 3, 4, 5A, and 5B. According to an embodiment of the present invention, the neutron beam plan may include the dose, irradiation time, irradiation intensity, irradiation angle, irradiation position, single field, multiple fields, medical images, conditions to start the irradiation, conditions to end the irradiation, and other information required by the neutron beam for the patient PT. The medical security management method 300 further includes, in process S3, the accelerator security control module 121 comprehensively determines whether to transmit and approve the first key 1 to the clinical security control module 112 based on the neutron beam generation request under the conditions of neutron beam plan, the neutron beam quality control (QC)/ quality assurance (QA) of the accelerator end 200, and the operation and inspection conditions of the accelerator end 200. Should the aforementioned condition meet the requirement, the process S5 is performed to transmit the first key 1 to the clinical security control module 112. Should the aforementioned condition not meet the requirement, the process S8 is performed to execute the process S4, denying the neutron beam generation request of the neutron beam plan, stopping the execution processes of the entire medical security management method 300.

According to an embodiment of the present invention, the medical security management method 300 further includes, in process S7, that the consensus mechanism module 130 determines whether the requirement of a default consensus mechanism is met or not by the received first key 1 and the second key 2. Should the aforementioned condition meet the requirement, then in process S9, the consensus mechanism module 130 allows the execution of the neutron beam plan based on the received first key 1 and the received second key 2, and drives the accelerator end 200 to generate the neutron beam. Should the aforementioned condition not meet the requirement, process S8 is performed to deny the neutron beam generation request of the neutron beam plan, and stop the execution processes of the entire medical security management method 300. In addition to the timestamp determination of the first key 1 and the second key 2 described in FIG. 2B, the consensus mechanism may also be, but not limited to digital signatures, encryption algorithms (which may be symmetric encryption or asymmetric encryption), and biometric technology (such as fingerprint, facial, or iris recognition). Therefore, this allows the further execution of identity verification with multiple control ends, resulting in the improvement of integration of multiple control ends during the execution of the medical security management method 300.

According to an embodiment of the present invention, the medical security management method 300 further includes, in process S10, that the monitoring module 113 and/or the accelerator security control module 121 accesses the monitoring data including the neutron beam generation status and/or the therapy status of the patient allowing the clinical security control module 112 to perform process S10a and process S10b based on the monitoring data. In process S10a and process S10b, the monitoring module 113 or the accelerator security control module 121 calculates and determines whether the dose of photons (e.g., gamma rays) and/or the dose of the neutron beam exceeds a default value, wherein the default value could be modified or defined by those skilled in the medical art based on the application needs. For instance, the dose of photons may followed by the regulations of the Nuclear Safety Commission of the Republic of China (Safety Standards for Ionizing Radiation Protection). In the regulations, the effective dose for radiation workers in any single year shall not exceed 50 millisieverts, and the equivalent dose to the eye lens shall not exceed 150 millisieverts within one year. Furthermore, the dose of the neutron beam is assessed by determining whether the cumulative dose delivered to the patient meets the criteria specified in the neutron beam treatment plan before, during, and after thereof, as well as the assessment of whether the patient's treatment condition remains within normal limits. In process S10a and process S10b, the monitoring module 113 or the accelerator security control module 121 determines whether the therapy status of the patient is normal. If the dose exceeds or the therapy status of the patient is abnormal, the process S8 is performed to deny the neutron beam generation request of the neutron beam plan, withdrawing the second key 2, and stopping the execution processes of the entire medical security management method 300. If the dose does not exceed or the therapy status of the patient is normal, which indicates that the neutron beam plan is still being performed, then process S6 is performed in process S11. The clinical control end 110 allows the second key 2 to be effective continually, thereby remaining the consensus mechanism effective in the consensus mechanism module 130, in order to generate and output the neutron beam through the accelerator end 200 continually.

In summary, the medical security system and method provided by the present invention establish an efficient and safe medical radiation therapy management mechanism through the clinical control end, neutron beam control end and consensus mechanism module. By introducing a consensus mechanism between the clinical control end and the neutron beam control end, ensuring that the neutron beam will be activated only when specific conditions in the consensus mechanism are met. The improvement overcomes the shortcomings of the interaction timing and communication between the two subsystems in the piror art, improving the integration and security of each terminal in the BNCT medical system, and the deployment flexibility of the system components at the near and far ends. It should be noticed that after reading the present invention, those skilled in the art may perform various modifications based on their needs; however, those modifications do not depart from the content intended to be protected within the scope of claims.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A medical security management system (100), **characterized by**, comprising:
a clinical security control module (112);
an accelerator security control module (121), configured to control the operation of an accelerator end (200); and
a consensus mechanism module (130), coupled to the clinical security control module (112) and the accelerator security control module (121), and configured to store at least one consensus mechanism;
wherein when the accelerator security control module (121) and the clinical security control module (112) transmit a first key (1) and a second key (2) to the consensus mechanism module (130), the consensus mechanism module (130) drives the accelerator end to generate neutron beam.

2. The medical security management system (100) according to claim 1, further comprising a neutron beam planning module (111), coupled to the accelerator security control module (121), and configured to formulate a neutron beam plan and transmit a neutron beam generation request to the accelerator security control module (121) to evaluate whether to transmit the first key (1).

3. The medical security management system (100) according to claim 2, wherein information of the neutron beam plan includes neutron beam dose, irradiation time, irradiation intensity, irradiation angle, irradiation position, single field, multiple fields, medical images, conditions to start the irradiation, conditions to end the irradiation, or any combination thereof.

4. The medical security management system (100) according to claim 1, wherein the accelerator security control module (121) is coupled to the clinical security control module (112), and the clinical security control module (112) transmits the second key (2) to the consensus mechanism module (130) based on the first key (1).

5. The medical security management system (100) according to claim 1, wherein one of the at least one consensus mechanisms comprises determining the neutron beam generation time according via the intersection of the time interval between the first key (1) and the second key (2).

6. The medical security management system (100) according to claim 1, wherein information carried by the first key (1) and the second key (2) includes at least one message related to communication of the timing that the neutron beam is generated, wherein the at least one message is selected from text, audio, video, symbols, digital codes, or any combination thereof.

7. The medical security management system (100) according to claim 1, wherein the consensus mechanism module (130) further includes a key verification unit (131) as an interactive identity verification between the first key (1) and the second key (2), and an identity verification approach is digital signatures, encryption algorithms, biometric technology, or any combination thereof.

8. The medical security management system (100) according to claim 1, further comprising a monitoring module (113), coupled to the clinical security control module (112) and the accelerator end (200), and configured to detect detection data, wherein the detection data includes a neutron beam generation status of the accelerator end (200) and a therapy status of a patient.

9. A medical security management method (300), **characterized by**, comprising:
preparing (S1) a neutron beam plan;
proposing (S2) a neutron beam generation request based on the neutron beam plan via a clinical control end (110);
transmitting (S5) a first key (1) to a consensus mechanism module (130) based on the neutron beam generation request via a neutron beam control end (120);
transmitting (S6) a second key (2) to the consensus mechanism module (130) via the clinical control end (110); and
performing a verification on the first key (1) and the second key (2) based on at least one consensus mechanism, and driving an accelerator end (200) to generate a neutron beam if the verification is passed via the consensus mechanism module (130).

10. The medical security management method (300) according to claim 9, wherein information of the neutron beam plan includes neutron beam dose, irradiation time, irradiation intensity, irradiation angle, irradiation position, single field, multiple fields, medical images, conditions to start the irradiation, conditions to end the irradiation, or any combination thereof.

11. The medical security management method (300) according to claim 9, wherein one of the at least one consensus mechanisms comprises determining the neutron beam generation time according via the intersection of the time interval between the first key (1) and the second key (2).

12. The medical security management method (300) according to claim 9, wherein information carried by the first key (1) and the second key (2) includes at least one message related to communication of the timing that the neutron beam is generated, wherein the at least one message is selected from text, audio, video, symbols, digital codes, or any combination thereof.

13. The medical security management method (300) according to claim 9, wherein the at least one consensus mechanism further includes an interactive identity verification between the first key (1) and the second key (2), and an identity verification approach is digital signatures, encryption algorithms, biometric technology, or any combination thereof.

14. The medical security management method (300) according to claim 9, further comprising:
accessing (S10) monitoring data via a monitoring module (113), wherein the monitoring data further includes a neutron beam generation status, including whether a neutron beam dose exceeds a default value,
when the default value is not exceeded, the second key (2) continues to be effective; and
when the default value is exceeded, the second key (2) is withdrawn by the clinical control end (110).

15. The medical security management method (300) according to claim 9, further comprising:
accessing (S10) monitoring data via a monitoring module (113), the monitoring data further includes a therapy status of a patient,
when the therapy status of the patient is normal, the second key (2) continues to be effective; and
when the therapy status of the patient is abnormal, the second key (2) is withdrawn by the clinical control end (110).

## Amended claims

### Amended claims in accordance with Rule 137(2) EPC.

1. A medical security management system (100), **characterized by**, comprising:
a clinical security control module (112);
an accelerator security control module (121), configured to control the operation of an accelerator end (200) to complete the generation of the neutron beam; and
a consensus mechanism module (130), coupled to the clinical security control module (112) and the accelerator security control module (121), and configured to store at least one consensus mechanism;
wherein when the accelerator security control module (121) and the clinical security control module (112) transmit a first key (1) and a second key (2) to the consensus mechanism module (130), the consensus mechanism module (130) drives the accelerator end to generate neutron beam;
wherein the first key (1) and the second key (2) include information and/or time.

2. The medical security management system (100) according to claim 1, further comprising a neutron beam planning module (111), coupled to the accelerator security control module (121), and configured to formulate a neutron beam plan and transmit a neutron beam generation request to the accelerator security control module (121) to evaluate whether to transmit the first key (1).

3. The medical security management system (100) according to claim 2, wherein information of the neutron beam plan includes neutron beam dose, irradiation time, irradiation intensity, irradiation angle, irradiation position, single field, multiple fields, medical images, conditions to start the irradiation, conditions to end the irradiation, or any combination thereof.

4. The medical security management system (100) according to claim 1, wherein the accelerator security control module (121) is coupled to the clinical security control module (112), and the clinical security control module (112) transmits the second key (2) to the consensus mechanism module (130) based on the first key (1).

5. The medical security management system (100) according to claim 1, wherein one of the at least one consensus mechanisms comprises determining the neutron beam generation time according via the intersection of the time interval between the first key (1) and the second key (2).

6. The medical security management system (100) according to claim 1, wherein information carried by the first key (1) and the second key (2) includes at least one message related to communication of the timing that the neutron beam is generated, wherein the at least one message is selected from text, audio, video, symbols, digital codes, or any combination thereof.

7. The medical security management system (100) according to claim 1, wherein the consensus mechanism module (130) further includes a key verification unit (131) as an interactive identity verification between the first key (1) and the second key (2), and an identity verification approach is digital signatures, encryption algorithms, biometric technology, or any combination thereof.

8. The medical security management system (100) according to claim 1, further comprising a monitoring module (113), coupled to the clinical security control module (112) and the accelerator end (200), and configured to detect detection data, wherein the detection data includes a neutron beam generation status of the accelerator end (200) and a therapy status of a patient.

9. A medical security management method (300), **characterized by**, comprising:
preparing (S1) a neutron beam plan;
proposing (S2) a neutron beam generation request based on the neutron beam plan via a clinical control end (110);
transmitting (S5) a first key (1) to a consensus mechanism module (130) based on the neutron beam generation request via a neutron beam control end (120);
transmitting (S6) a second key (2) to the consensus mechanism module (130) via the clinical control end (110); and
performing a verification on the first key (1) and the second key (2) based on at least one consensus mechanism, and driving an accelerator end (200) to complete the generation of a neutron beam if the verification is passed via the consensus mechanism module (130);
wherein the first key (1) and the second key (2) include information and/or time.

10. The medical security management method (300) according to claim 9, wherein information of the neutron beam plan includes neutron beam dose, irradiation time, irradiation intensity, irradiation angle, irradiation position, single field, multiple fields, medical images, conditions to start the irradiation, conditions to end the irradiation, or any combination thereof.

11. The medical security management method (300) according to claim 9, wherein one of the at least one consensus mechanisms comprises determining the neutron beam generation time according via the intersection of the time interval between the first key (1) and the second key (2).

12. The medical security management method (300) according to claim 9, wherein information carried by the first key (1) and the second key (2) includes at least one message related to communication of the timing that the neutron beam is generated, wherein the at least one message is selected from text, audio, video, symbols, digital codes, or any combination thereof.

13. The medical security management method (300) according to claim 9, wherein the at least one consensus mechanism further includes an interactive identity verification between the first key (1) and the second key (2), and an identity verification approach is digital signatures, encryption algorithms, biometric technology, or any combination thereof.

14. The medical security management method (300) according to claim 9, further comprising:
accessing (S10) monitoring data via a monitoring module (113), wherein the monitoring data further includes a neutron beam generation status, including whether a neutron beam dose exceeds a default value,
when the default value is not exceeded, the second key (2) continues to be effective; and
when the default value is exceeded, the second key (2) is withdrawn by the clinical control end (110).

15. The medical security management method (300) according to claim 9, further comprising:
accessing (S10) monitoring data via a monitoring module (113), the monitoring data further includes a therapy status of a patient,
when the therapy status of the patient is normal, the second key (2) continues to be effective; and
when the therapy status of the patient is abnormal, the second key (2) is withdrawn by the clinical control end (110).
